# EUROPEAN PATENT APPLICATION

(11) **EP 4 606 901 A1**
(43) Date of publication of application: **27.08.2025**
(21) Application number: 24746607.1
(22) Date of filing: 02.01.2024
(51) Int. Cl.: C12N 15/85, C12N 15/13, A01K 67/0278, C12N 5/10, C07K 16/28, C07K 16/00, A01K 67/0275

(54) **ANIMAL MODEL FOR PRODUCING HUMANIZED ANTIBODY, AND CONSTRUCTION METHOD THEREFOR**

(71) Applicant: Gempharmatech Co., Ltd, Nanjing, Jiangsu 210061 (CN)
(72) Inventor: ZHANG, Mingkun, Nanjing Jiangsu 210061 (CN); JU, Cunxiang, Nanjing Jiangsu 210061 (CN); ZHANG, Yuxi, Nanjing Jiangsu 210061 (CN); LI, Song, Nanjing Jiangsu 210061 (CN); ZHAO, Jing, Nanjing Jiangsu 210061 (CN); GAO, Xiang, Nanjing Jiangsu 210061 (CN)
(74) Representative: Gong, Jinping
(86) International application number: PCT/CN2024/070020
(87) International publication number: WO 2025/145264

(57) **Abstract**

The present application relates to a non-human animal and a method for preparing the non-human animal. The non-human animal is operably linked to a human immunoglobulin variable region gene downstream of an immunoglobulin locus. The present application further provides a method for generating an antibody through the non-human animal.

## Description

### TECHNICAL FIELD

The present application relates to the field of biomedicines, in particular to a non-human animal model generating antibodies and a construction method thereof.

### BACKGROUND

Therapeutic monoclonal antibody drugs have become one of the most important components of biomedicines through more than thirty years of development. They represent a class of rapidly growing and most advanced and effective drugs. Since OKT3 was first approved in 1986, the development history of therapeutic antibodies has witnessed rapid and revolutionary progress, consolidating its position as an important aspect of modern biopharmaceuticals. Despite these successes are gained, challenges still exist, including low success rates, high costs, and long time for antibody-based drug development. In recent years, the issue of immunogenicity induced anti-drug antibodies (ADA) has also caused grave concerns, especially for therapeutic antibodies classified as macromolecular drugs. Overcoming the risk of ADA in the pre-clinical stage still remains a complex task.

At present, a commonly used method for discovering and screening of therapeutic monoclonal antibodies is the "display technology", including phage display, bacterial display, yeast display and the like. The "display technology" can be used for rapidly screening antibodies. However, the diversity and affinity of the antibodies screened by these methods are limited due to the lack of in-vivo processes such as antibody variable region recombination and affinity maturation. So a large amount of costs and cycles are often required for further optimization and modification. Another commonly used method for discovering and screening of therapeutic monoclonal antibodies is to directly use wild-type animal models for immune detection, such as poultry, rodents and non-human primates. The molecules obtained by the above methods are subjected to humanization modification based on experience and computer database prediction, which is a commonly used method to avoid an ADA effect caused by immunogenicity. However, its final modification result is often unpredictable and disconnected from the effect of clinical practice, leading to adverse effects such as allergic stress and loss of efficacy in neutralizing anti-antibodies during clinical treatment.

A possible method to avoid the above issues and discover fully human antibodies is to use engineered non-human animals that carry human antibody genes. At present, although many available non-human animals can produce fully human antibodies, these models often have some major drawbacks or limitations due to the complexity and complicacy of the human antibody coding lineage. As they often only contain very few or some human antibody lineages, the lack of the human antibody lineages means a lack of diversity in the ultimately discovered antibody molecules and a decrease in the likelihood of obtaining high affinity. And what followed is a relatively low V(D)J recombination efficiency, and the yield of antibody is below a normal level. In addition, there are also issues such as immunogenicity caused by endogenous antibody coding infiltration from animals themselves, as well as gene defect phenotypes caused by large fragment deletion of endogenous genes. Due to the existence of the above issues and some other issues, there is a need for a non-human animal comprising fully human immunoglobulin variable region coding genes and having the ability of producing humanized antibodies without change in immune response. To this regard, such an animal would enable the effective and efficient production of clinical variable region fully humanized antibodies.

### SUMMARY

The present application provides a non-human animal and a method for preparing the non-human animal. The genome of the non-human animal comprises a human immunoglobulin variable region gene operably linked downstream of an immunoglobulin locus The non-human animal of the present application can avoid interfering with gene expression and regulation of animals themselves and prevent the leakage of endogenous variable region genes, and has significant advantages in antibody production.

In one aspect, the present application provides a method for preparing the non-human animal, the method comprising operably linking the human immunoglobulin variable region gene downstream of the immunoglobulin locus of the non-human animal.

In some embodiments, the method comprises operably linking a human immunoglobulin heavy chain variable region gene downstream of the heavy chain locus of the non-human animal. In some embodiments, the method comprises operably linking one or more human heavy chain V, D or J regions or fragments thereof downstream of the heavy chain constant region locus of the non-human animal. In some embodiments, the genes of the more human heavy chain V, D or J regions or fragments thereof can be recombined. In some embodiments, the genes of the more human heavy chain V, D or J regions, or fragments thereof are directly linked.

In some embodiments, the method comprises operably linking a human immunoglobulin light chain variable region gene downstream of the light chain locus of the non-human animal. In some embodiments, the method comprises operably linking one or more human light chain V, D or J regions or fragments thereof downstream of the light chain constant region locus of the non-human animal. In some embodiments, the genes of the more human light chain V, D or J regions or fragments thereof can be recombined. In some embodiments, the genes of the more human light chain V or J regions or fragments thereof are directly linked.

Therefore, in another aspect, the present application provides a method for preparing the non-human animal, the method comprising operably linking a human immunoglobulin heavy chain variable region gene downstream of the immunoglobulin heavy chain locus of the non-human animal.

In another aspect, the present application provides a method for preparing a non-human animal, the method comprising operably linking a human immunoglobulin light chain variable region gene downstream of the immunoglobulin light chain locus of the non-human animal.

In one aspect, the present application provides a non-human animal or a non-human animal cell, wherein the genome of the non-human animal or the non-human animal cell comprises a human immunoglobulin variable region gene operably linked downstream of the immunoglobulin locus. The following involved features that are contained of the non-human animal are all included of the non-human animal cell.

In some embodiments, a human immunoglobulin heavy chain variable region gene is operably linked downstream of the immunoglobulin heavy chain locus of the non-human animal. In some embodiments, the genes of one or more human heavy chain variable V, D or J regions or fragments thereof are operably linked downstream of the heavy chain constant region locus of the non-human animal. In some embodiments, the genes of the more human heavy chain V, D or J regions, or fragments thereof can be recombined. In some embodiments, the genes of the more human heavy chain V, D or J regions, or fragments thereof are directly linked.

In some embodiments, a human immunoglobulin light chain variable region gene is operably linked downstream of the light chain locus of the non-human animal. In some embodiments, the genes of one or more human light chain V or J regions or fragments thereof are operably linked downstream of the light chain constant region locus of the non-human animal. In some embodiments, the genes of the more human light chain Vor J regions, or fragments thereof can be recombined. In some embodiments, the genes of the more human light chain V or J regions, or fragments thereof are directly linked.

Therefore, in another aspect, the present application provides a non-human animal or a non-human animal cell. A human immunoglobulin heavy chain variable region gene is operably linked downstream of the immunoglobulin heavy chain locus of the non-human animal or the non-human animal cell.

In another aspect, the present application also provides the offsprings of the non-human animal. The offsprings can be offsprings generated by mating the non-human animal with the same genotype or other genotypes.

In another aspect, the present application also provides a cell derived from the non-human animal or offsprings thereof (for example, stem cells, embryonic stem cells, immune cells, B cells, T cells, or hybridomas), or cell lines or primary cell cultures thereof.

In another aspect, the present application provides a non-human animal or a non-human animal cell. A human immunoglobulin light chain variable region gene is operably linked downstream of the immunoglobulin light chain locus of the non-human animal or the non-human animal cell.

In some embodiments, the non-human animal comprises endogenous immunoglobulin variable region genes. In some embodiments, the integrity of the endogenous immunoglobulin variable region gene of the non-human animal is not damaged. In some embodiments, the function of the expression regulatory element of the endogenous immunoglobulin variable region gene of the non-human animal is not damaged. In some embodiments, the endogenous immunoglobulin variable region gene of the non-human animal comprises a functional protein encoding gene and a functional microRNA gene. In some embodiments, the non-human animal comprises an expression regulatory element of an intact endogenous immunoglobulin variable region. In some embodiments, the non-human animal comprises an intact endogenous immunoglobulin variable region gene.

In some embodiments, the non-human animal does not express the endogenous immunoglobulin variable region. In some embodiments, the endogenous immunoglobulin variable region gene of the non-human animal does not express the functional protein.

In some embodiments, the transcription direction of the human immunoglobulin variable region gene of the non-human animal is opposite to that of the endogenous immunoglobulin variable region gene.

In some embodiments, the transcription direction of the immunoglobulin constant region gene of the non-human animal is opposite to that of the endogenous immunoglobulin variable region gene. The immunoglobulin constant region gene of the non-human animal can be endogenous. The non-human animal cannot comprise an exogenous immunoglobulin constant region gene.

In some embodiments, the transcription direction of the human immunoglobulin variable region gene of the non-human animal is the same as that of the immunoglobulin constant region gene of the non-human animal.

In some embodiments, a heavy chain constant region gene is contained between the human immunoglobulin heavy chain variable region gene and the endogenous immunoglobulin heavy chain variable region gene of the non-human animal. In some embodiments, one or more heavy chain constant region genes are contained between the human immunoglobulin heavy chain variable region gene and the endogenous immunoglobulin heavy chain variable region gene of the non-human animal. In some embodiments, a distance between the human immunoglobulin heavy chain variable region gene and the endogenous immunoglobulin heavy chain variable region gene of the non-human animal is 169 Kbp-240 Kbp.

In some embodiments, a light chain constant region gene is contained between the human immunoglobulin light chain variable region gene and the endogenous immunoglobulin light chain variable region gene of the non-human animal. In some embodiments, one or more light chain constant region genes are contained between the human immunoglobulin light chain variable region gene and the endogenous immunoglobulin light chain variable region gene of the non-human animal. In some embodiments, a distance between the human immunoglobulin light chain variable region gene and the endogenous immunoglobulin light chain variable region gene of the non-human animal is 4 Kbp-42 Kbp.

In some embodiments, the non-human animal is a mammal. In some embodiments, the non-human animal is a rodent. In some embodiments, the non-human animal is a mouse. In some embodiments, the non-human animal is a vole.

In some embodiments, the human immunoglobulin heavy chain variable region gene is inserted between Tmem121 and Igha genes at the locus of the mouse. In some embodiments, the human immunoglobulin heavy chain variable region genes are inserted among mouse chromosome positions chr12:113,149,523-113,223,857. In some embodiments, the human immunoglobulin heavy chain variable region genes are inserted among mouse chromosome positions chr12:113,190,256.

In some embodiments, the human immunoglobulin light chain variable region gene is inserted between lgkc and Rpia genes at the locus of the mouse. In some embodiments, the human immunoglobulin light chain variable region genes are inserted among mouse chromosome positions chr6: 70,703,738-70,742,704. In some embodiments, the human immunoglobulin light chain variable region genes are inserted among mouse chromosome positions chr6:70,706,267.

In some embodiments, the method comprises operably linking the human immunoglobulin variable region gene downstream of the immunoglobulin locus of the non-human animal through site-directed recombination. In some embodiments, the site-directed recombination can be performed once or many times. In some embodiments, the method comprises linking genes of multiple human heavy chain V, D or J regions or fragments thereof by using many times of site-directed recombination. In some embodiments, the method comprises linking genes of multiple human light chain V or J regions or fragments thereof by using many times of site-directed recombination. In some embodiments, the site-directed recombination comprises: a homologous recombination method, a nucleic acid cleavage enzyme mediated recombination method and a site-specific recombination enzyme mediated recombination. In some embodiments, the human immunoglobulin variable region gene is inserted into the downstream of the immunoglobulin locus of the non-human animal by using a vector carrying a human genome fragment. In some embodiments, the human immunoglobulin variable region gene is inserted into the downstream of the immunoglobulin locus of the non-human animal by using a site-specific recombination system mediated site-directed integration method. In some embodiments, the method comprises deleting a resistance marker after the insertion of the human immunoglobulin variable region gene.

In some embodiments, the method comprises allowing the transcription direction of the immunoglobulin constant region gene of the non-human animal to be opposite to that of the endogenous immunoglobulin variable region gene through chromosome recombination before the insertion of the human variable region gene. In some embodiments, the method comprises reversing the transcription direction of the immunoglobulin constant region gene of the non-human animal through chromosome recombination before the insertion of the human variable region gene. In some embodiments, the chromosome recombination comprises site-specific recombination system mediated genome fragment reversion. In some embodiments, the method comprises deleting a resistance marker after chromosome recombination.

In another aspect, the present application provides a non-human animal cellwhose genome comprises a human immunoglobulin variable region gene operably linked downstream of the immunoglobulin locus. In some embodiments, the cell is an embryonic stem (ES) cell. In some embodiments, the cell is a reproductive cell. In some embodiments, the cell is a fertilized egg cell. In some embodiments, the cell is an embryonic cell.

In some embodiments, the non-human animal cell can be developed into a non-human animal.

In another aspect, the present application provides cells, tissues and organs derived from the non-human animal.

In another aspect, the present application provides a method for preparing an antibody specifically binding to an antigen, the method comprising immunizing the non-human animal with the antigen.

In another aspect, the present application provides a method for preparing an antibody specifically binding to an antigen, the method comprising: exposing the non-human animal or cell into the antigen, and generating hybridomas from the cells collected from the animal. The method can also comprise collecting the chimeric antibody generated by the hybridomas. The method can further comprise sequencing the variable region gene of the hybridoma.

In another aspect, the present application provides a method for preparing an antibody specifically binding to an antigen, the method comprising: exposing the non-human animal or cell into the antigen, and sequencing the nucleic acids encoding human heavy chain and light chain immunoglobulin variable regions in the animal or cell. The non-human animal or cell can express a chimeric antibody specifically binding to the antigen. In one embodiment, the method can comprise operably linking a nucleic acid encoding the human heavy chain immunoglobulin variable region to a nucleic acid encoding the heavy chain immunoglobulin constant region, and expressing the antibody specifically binding to the antigen. The constant region can be a human heavy chain constant region, or can be a heavy chain constant region of the non-human animal. In one embodiment, the method can comprise operably linking a nucleic acid encoding the human light chain immunoglobulin variable region to a nucleic acid encoding the light chain immunoglobulin constant region, and expressing the antibody specifically binding to the antigen. The constant region can be a human light chain constant region, or can be a light chain constant region of the non-human animal.

In another aspect, the present application provides a method for preparing a sample, the method comprises: exposing the non-human animal of the present application into an antigen; and collecting the sample from the non-human animal. In some embodiments, the sample comprises an immune cell, for example a B cell. In some embodiments, the sample comprises bone marrow, spleen tissues, lymph nodes, spleen cells or peripheral lymphocytes.

The technical scheme of the present application comprises the following one or more advantages:
The non-human animal of the present application has the advantages in the aspects of avoiding interfering with gene expression and regulation of animals themselves. For example, a human variable region gene (for example, a light chain variable region, a heavy chain variable region, or the light chain variable region and the heavy chain variable region) is inserted downstream (or in the rear) of the endogenous immunoglobulin locus of the mouse cell, insertion of a large fragment of DNA sequence or deletion of DNA sequence is avoided in the endogenous immunoglobulin locus of the non-human animal (for example, a mouse). Since there is a very long "Junk sequence" downstream of the endogenous immunoglobulin locus of the non-human animal (for example, a mouse), with reference to a recently disclosed GRCm39 mouse full-genome sequence, there is a sequence having a length of up to 66 kb bases and no functional gene report downstream (in the rear) of the mouse immunoglobulin heavy chain gene, and there is a sequence having a length of up to 39 kb and no functional gene report downstream (in the rear) of the mouse immunoglobulin κ light chain gene. The introduction of exogenous large fragment genes in these long "Junk sequences" can avoid interfering with the expression and regulation of the endogenous gene of the non-human animal (such as a mouse) at the genomic level.

The non-human animal of the present application also has the advantages in avoiding interfering with the expression and regulation of the animal itself. For example, the modification or deletion of the immunoglobulin variable region gene of the non-human animal (for example, a mouse) is avoided. Since there are functional protein coding genes and functional microRNA genes inside the immunoglobulin variable region locus of the non-human animal (for example, a mouse), the deletion or modification of these genes may cause unpredictable phenotypes appearing in animals themselves. For example, a functional protein coding gene *Adam6a* is contained inside the immunoglobulin variable region locus in the mouse, and the modification and deletion of the *Adam6a* gene can cause male infertility in mice.

The non-human animal of the present application has the advantages in antibody production. In the non-human animal of the present application, the immunoglobulin variable region genes and constant region genes of the host non-human animal are both retained, i.e., the endogenous immunoglobulin variable region genes and constant region genes of the non-human animal are both retained. Where, all the host immunoglobulin regulatory sequences comprise promoters, enhancers, transition regions and other potential expression regulatory sequences which are all retained. Therefore, immunoglobulin gene recombination and expression and B cell development and affinity maturation of the non-human animal can be better ensured, etc.

The non-human animal of the present application has the advantages in antibody production. For example, in some embodiments, the endogenous immunoglobulin constant region in the animal is completely or partially modified (for example, the constant region is completely or partially reversed), so that its transcription direction is opposite to that of the endogenous immunoglobulin variable region gene. It is avoided that V(D)J recombination occurs in the endogenous variable region and the endogenous constant region to generate the antibody.

The non-human animal of the present application also has the advantages in antibody production. For example, in some embodiments, the inserted exogenous human immunoglobulin variable region is far away from the endogenous variable region, and their transcription directions are opposite. It is avoided that the endogenous variable region is integrated into human variable region VDJ recombination or VJ recombination due to DNA recombinase modification and recombination, and then it is highly likely to avoid the production of antibodies with immunogenic epitopes in human caused by an endogenous variable region lineage.

The non-human animal of the present application also has the advantages in antibody production. For example, in some embodiments, all the fragments of the human variable region gene are gradually introduced and inserted downstream (in the rear) of the endogenous immunoglobulin locus of the non-human animal (for example, a mouse) cell, the variable domain generated by the obtained non-human animal can have diversity that is almost the same as that of the variable domain in human. This also means that the obtained non-human animal has antibody diversity as close as possible to that of human body itself and the probability to obtain a high-affinity specific antibody.

The method for preparing the non-human animal according to the present application also has the advantages. For example, in some schemes, the endogenous immunoglobulin constant region of the non-human animal (for example, a mouse) is completely or partially modified so that its transcription direction is opposite to that of the endogenous variable region gene. Then, the modified human variable region gene is inserted downstream (in the rear) of the endogenous immunoglobulin locus of the non-human animal (for example, a mouse) cell, and the human variable region gene can be segmented from back to front and is inserted in a direction opposite to the transcription direction of the immunoglobulin locus in the animal. The resulting effects are that almost every modified cell of the partial human variable region obtained by insertion is injected to early animal embryos to prepare a chimeric animal (for example, a mouse), i.e., a non-human animal generated or obtained by breeding. The obtained non-human animals can be subjected to VDJ or VJ recombination by utilizing the inserted partial human variable region gene, and generate a chimeric antibody with a functional human variable region. These non-human animals carrying partial human variable region can be used as check points for preparing non-human animals finally having complete human variable regions. The reliability and functionality of segmented insertion of human variable regions are judged from the perspective of antibody production and diversity of variable region domains, and success or failure prediction and route correction of a non-human animal having complete human variable regions are made timely. These non-human animals carrying partial human variable regions can also be discovered by using an antibody for production of chimeric antibodies with human variable regions.

Those skilled in the art can readily perceive other aspects and advantages of the present application from the detailed description below. The following detailed descriptions merely show and describe the exemplary embodiments of the present application. As will be recognized by those skilled in the art, the contents of the present application enable those skilled in the art to make changes to disclosed specific embodiments without departing from the inventive spirit and scope of the present application. Correspondingly, the drawings of the present application and the descriptions of the specification are only illustrative, but not limiting.

### BRIEF DESCRIPTION OF THE DRAWINGS

Specific features involved in the present application are as shown in appended claims. The features and advantages of the present application will be better understood by reference with exemplary embodiments and advantages described in detail hereinafter. The brief explanations of accompanying drawings are as follows:
FIG. 1 shows a heavy chain Igh constant region undergoes Cpoint and LD targeting as well as C region modification.
FIG. 2 is a diagram showing human heavy chain variable region insertion, comprising insertion targeting and resistance marker deletion.
FIG. 3 shows a light chain Igk constant region undergoes Cpoint and LD targeting as well as C region modification.
FIG. 4 is a diagram showing human light chain variable region insertion, comprising insertion targeting and resistance marker deletion.
FIG. 5 shows polymerase chain reaction (PCR) identification results of Cpoint targeting.
FIG. 6 shows PCR identification results of LD targeting.
FIG. 7 shows reverse PCR identification results.
FIG. 8 shows PCR identification results of resistance marker deletion.
FIG. 9 shows PCR identification results of IGH vector1 targeting.
FIG. 10 shows PCR identification results of IGH vector1 target integrity.
FIG. 11 shows PCR identification results of IGH vector1 resistance marker deletion.
FIG. 12 shows an availability test of an IGH insertion gene.
FIG. 13 shows an availability test of an IGK insertion gene.
FIG. 14 shows that lymphocytes and myeloid cells in humanized mouse spleen are in normal proportion.
FIG. 15 shows that B cells in spleen and bone marrow are normally developed.
FIG. 16 shows leakage expression of mouse derived genes after different mouse regions are reversed.
FIG. 17 shows enzyme linked immunosorbent assay (ELISA) titer detection of a humanized mouse and a control mouse.
FIG. 18 shows comparison of drug efficacy between screened 10K4F3 antibody and a marketed drug in mice.
FIG. 19 shows an availability test of partial IGH-V, F and J insertion genes.
FIG. 20 shows an availability test of partial IGH-V and J insertion genes.
FIG. 21 shows ELISA titer detection of a partial VDJ humanized mouse and a control mouse (S0, before immunization; S1, secondary immunization; S2, triple immunization).

### DETAILED DESCRIPTION

Next, the embodiments of the present application will be illustrated through specific examples, other advantages and effects of the present application can be easily known by those skilled in the art according to contents disclosed in this specification.

### Term definitions

In the present application, the term "non-human animal" generally refers to all non-human vertebrates, for example mammals and non-mammals, such as non-human primates, rodents, rabbits, camels, sheep, dogs, cats, horses, cows, birds, amphibians and reptiles. For example, the non-human animal can be a rat, or a mouse.

In the present application, the term "constant region" generally refers to a sum of domains of an antibody except variable regions. The constant region does not directly involve the binding of an antigen, but shows different effector functions. Depending on the amino acid sequences of the constant regions of their heavy chains, the antibodies are classified as the following categories: IgA, IgD, IgE, IgG and IgM, and some of them can be further classified as categories IgG1, IgG2 and IgG3, as well as IgG4, IgA1 and IgA2. The heavy chain constant regions corresponding to different categories of antibodies are referred to as α, δ, ε, γ and µ, respectively. The light chain constant regions found in all of 5 categories of antibodies are referred to as κ (kappa) and λ (lambda). The genes encoding mouse (Mus musculus) constant regions can comprise IGHA, IGHD, IGHE, IGHG1, IGHG2A, IGHG2B, IGHG2C, IGHG3 or IGHM. Information about mouse constant region loci can see IMGT Repertoire: https://www.imgt.org/IMGTrepertoire/index.php?section=LocusGenes&repertoire=genetable &species=Mus_musculus&group=IGHC.

In the present application, the term "locus" generally refers to a specific region along a chromosome or a DNA sequence. Depending on the contexts, the locus can be a specific sequence of a gene, a marker, a chromosome band, or one or more nucleotides. In the present application, when it comes to the immunoglobulin locus, the locus comprises a genetic element or a set of related genetic elements whose cells can be used for expressing the information of an immunoglobulin peptide. As to a locus that is not rearranged, the genetic element can be assembled by a B cell precursor to form a gene encoding the immunoglobulin peptide. As to a locus that is rearranged, the gene encoding the immunoglobulin peptide is contained in the locus.

In the present application, the term "antibody" generally refers to a support or skeleton portion comprising an intact antibody or an antigen binding fragment thereof, and optionally allowing a portion binding to the antigen to adopt a conformation promoting the binding of the antibody to the antigen. The examples of the antibody include but are not limited to a monoclonal antibody, a polyclonal antibody, Fab, Fab', F(ab)2, a Fv fragment, F(ab')2, scFv, di-scFv and/or dAb, an immunoconjugate, a multi-specific antibody (for example a bispecific antibody), an antibody fragment, an antibody derivative, an antibody analogue or a fusion protein and the like, as long as they show required antigen binding activity. The term also includes a genetically engineered antibody such as a chimeric antibody (for example a humanized mouse antibody), a humanized antibody, a fully human antibody and a heterologous covalent antibody (for example a bispecific antibody).

In the present application, the term "chimeric antibody" generally refers to an antibody comprising sequences present in at least two different antibodies (for example, antibodies derived from two different mammal species, for example human and mouse antibodies). The non-limiting examples of the chimeric antibody are antibodies comprising variable domain sequences (for example, all or a part of light chain variable domain and/or heavy chain variable domain sequences) of a human antibody and constant domains of a non-human antibody.

In the present application, "immunoglobulin" generally refers to a protein essentially consisting of one or more polypeptides encoded by immunoglobulin genes. The recognized human immunoglobulin genes comprise κ, λ, α (IgA1 and IgA2), γ (IgG1, IgG2, IgG3, and IgG4), δ, ε and µ constant region genes, as well as numerous immunoglobulin variable region genes. The NH2- terminal (about 110 amino acids) of a full-length immunoglobulin "light chain" (about 25 KD and 214 amino acids) is encoded by variable region genes, and the COOH- terminal is encoded by κ or λ constant region genes. The full-length immunoglobulin "heavy chain" (about 50 KD and 446 amino acids) is encoded similarly by variable region gene (about 116 amino acids) and one of the above other constant region genes such as γ (encoding about 330 amino acids). The term "immunoglobulin" comprises immunoglobulins of CDR from human or non-human sources. The structure of the immunoglobulin can be human, humanized or non-human, for example a mouse structure with reduced antigenicity in a human body via modification, or a synthesized structure for example a consensus sequence.

In the present application, the term "variable region" generally refers to a region where an antibody molecule binds to a specific antigen. It consists of antigen binding sites of a heavy chain and a light chain. The variable regions are different between different B cell immunoglobulins, but are the same between all the immunoglobulins produced by the same B cell. The diversity of the variable region is generated by gene recombination of variable region genes during the B cell maturation. This process is a rearrangement process which generates lots of diversity capable of binding to any given antigens, and therefore an immune system recognizes and neutralizes a large amount of antigenic burdens caused by exogenous and pathogenic structures. Therefore, an antibody library is composed of rich immunoglobulins having different V regions, but the immunoglobulins have the same Fc portion.

In the present application, the term "endogeneity" generally refers to any substance derived from or within organisms, cells, tissues, or systems. For example, an endogenous gene, i.e., a gene naturally occurring in a non-human animal, is different from an exogenous gene that is from other animals, and introduced through transgenosis.

In the present application, the terms "upstream" and "downstream" are generally descriptions of relative positions between genes in a chromosome, with reference to the chromosome, or a given gene. In the present application, to better conclude modification common points of heavy chain and light chain loci, with reference to natural immunoglobulin locus position of a non-human animal host and its immunoglobulin transcription direction (i.e., as shown in NCBI database), for example, for gene A, if the position of the gene A is the rear of the coding terminal of the immunoglobulin locus relative to the host immunoglobulin locus, it is considered that A is located downstream of the host immunoglobulin locus, and the host immunoglobulin locus is located upstream of A.

In the present application, the term "transcription direction" generally refers to a gene coding direction where genes finally form a functional protein, i.e., a direction from a sequence of a coding initiation codon (such as ATG) to a sequence of a coding termination codon (such as TAG, TAA and TGA).

### Detailed embodiments

The present application relates to a genetically modified non-human animal and cell that can generate variable regions as chimeric antibodies of a human and has functional human immunoglobulin variable region genes. In the present application, the researches for mice are only for illustrative purposes, unless otherwise specified, regarding mice, also includedare all non-human mammals, and mice are preferred non-human mammals.

In one aspect, the non-human animal or cell involved in the present application has one or more human IGHV regions, one or more human IGHD regions and/or one or more human IGHJ regions downstream of the heavy chain locus coding region of the host non-human mammal. In some embodiment, the transcription direction of the coding region is opposite to that of the coding region of the endogenous heavy chain VDJ protogene in the host after the human IGHV region, the human IGHD region and/or the human IGHJ region is inserted. The human IGHV region, human IGHD region and the human IGHJ region are operably linked, and can be subjected to VDJ recombination.

In some embodiments, the constant region of the endogenous heavy chain locus in the animal is completely or partially modified, so that its transcription direction is completely or partially opposite to the transcription direction of the endogenous heavy chain variable region gene.

In another aspect, the non-human animal or cell of the present application can have one or more human light chain (such as IGK) V regions and/or one or more human light chain (such as IGK) J regions downstream of the light chain locus coding region of the hostnon-human animal. In some embodiments, the transcription direction of the coding region is opposite to the transcription direction of the coding region of the corresponding endogenous light chain VJ protogene in the host after the human light chain (such as IGK) V region and the human light chain (such as IGK) J region are inserted. The human IGKV region and the human IGKJ region are operably linked, and can be subjected to VJ recombination.

In some embodiments, the constant region of the endogenous light chain locus in the animal is completely or partially modified, so that its transcription direction is completely or partially opposite to the transcription direction of the corresponding endogenous light chain variable region gene.

In one aspect, the human immunoglobulin heavy chain VDJ region gene located downstream of the heavy chain locus coding region of the host non-human animal comprises all reversed arranged heavy chain V, D and J regions from human in a manner of germline as well as partial or complete interleaved sequences.

In another aspect, the human immunoglobulin heavy chain VDJ region genes located downstream of the heavy chain locus coding region of the host non-human animal are reversely arranged and can be operably linked to the modified host constant regions, and are accessibly subjected to VDJ recombination and expression of functional human variable region chimeric antibodies.

In another aspect, the reversely arranged human immunoglobulin genes located downstream of the heavy chain locus coding region of the host non-human animal can be expressed with different antibody isotypes of constant region compositions, and are accessibly subjected to immunoglobulin isotype switching of B cells.

In one aspect, the human immunoglobulin light chain VJ region gene located downstream of the light chain locus coding region of the host non-human animal comprises all reversed arranged light chain V and J regions from human in a manner of germline as well as partial or complete interleaved sequences.

In another aspect, the human immunoglobulin light chain VJ region genes located downstream of the light chain locus coding region of the host non-human animal are reversely arranged, can be operably linked to the modified host constant regions, are accessibly subjected to VJ recombination and express functional human variable region chimeric antibodies.

In one aspect, the inserted human immunoglobulin variable region gene DNA comprises 10%-100% of reversely encoded human heavy chain variable V region gene coding sequences, such as over 60%, over 70%, over 80%, over 90% and all of the human heavy chain variable region gene coding region.

In one aspect, the inserted human immunoglobulin variable region gene DNA comprises 50%-100% of reversely encoded human heavy chain D region gene coding sequences, such as over 60%, over 70%, over 80%, over 90% and all of the human D region gene coding region.

In one aspect, the inserted human immunoglobulin variable region gene DNA comprises 50%-100% of reversely encoded human heavy chain J region gene coding sequences, such as over 60%, over 70%, over 80%, over 90% and all of the human J region gene coding region.

In one aspect, the length of the inserted human immunoglobulin heavy chain variable region gene DNA is about or at least 10 kb, 20 kb, 30 kb, 40 kb, 50 kb, 60 kb, 70 kb, 80 kb, 90 kb, 100 kb, 200 kb, 300 kb, 400 kb, 500 kb, 600 kb, 700 kb, 800 kb, 900 kb, 1000 kb, 1500 kb, 2000 kb, 2500 kb, 3000 kb or 3500 kb.

In one aspect, the inserted human immunoglobulin variable region gene DNA comprises 15%-100% of reversely encoded human light chain V region gene coding region, such as over 60%, over 70%, over 80%, over 90% and all of the human V region gene coding region.

In one aspect, the inserted human immunoglobulin variable region gene DNA comprises 50%-100% of reversely encoded human light chain J region gene coding region, such as over 60%, over 70%, over 80%, over 90% and all of the human J region gene coding region.

In one aspect, the length of the inserted human immunoglobulin heavy chain variable region gene DNA is about or at least 10 kb, 20 kb, 30 kb, 40 kb, 50 kb, 60 kb, 70 kb, 80 kb, 90 kb, 100 kb, 200 kb, 300 kb, 400 kb, 500 kb, 600 kb, 700 kb, 800 kb, 900 kb, 1000 kb, 1500 kb, 2000 kb, 2500 kb, 3000 kb or 3500 kb.

Information about human heavy chain V, D and J regions as well as light chain V and J regions can refer to IMGT Repertoire: https://www.imgt.org/IMGTrepertoire/LocusGenesl.

Optionally, the non-human animal can only contain a human heavy chain variable region gene located downstream of the heavy chain locus coding region of the host non-human animal, or can only contain a human light chain variable region gene located downstream of the light chain locus coding region of the host non-human animal.

Optionally, any modification or deletion of the endogenous immunoglobulin heavy chain variable region (V, D and J regions) genes of the non-human animal is not performed to prevent the expression or regulation of some genes of the host in the variable region from being affected. Meanwhile, the endogenous immunoglobulin heavy chain variable region is far away from the inserted human immunoglobulin heavy chain variable region, and an endogenous immunoglobulin heavy chain constant region coding sequence is spaced between them. A distance between them is a base length ranging from169 Kbp to 240 Kbp, preferably, 202 Kbp. To a certain extent, it is avoided that the endogenous variable region is permeated into the final chimeric immunoglobulin coding gene recombination.

Optionally, any modification or deletion of the endogenous immunoglobulin light chain variable region (V and J regions) genes of the non-human animal is not performed to prevent the expression or regulation of some genes of the host in the variable region from being affected. Meanwhile, the endogenous immunoglobulin light chain variable region is far away from the inserted human immunoglobulin light chain variable region, and an endogenous immunoglobulin light chain constant region coding sequence is spaced between them. A distance between them is a base length ranging from 4 Kbp to 42 Kbp, preferably, 5 Kbp. To a certain extent, it is avoided that the endogenous variable region is permeated into the final chimeric immunoglobulin coding gene recombination.

The present application discloses a method for constructing a human immunoglobulin variable region gene with functionality in a non-human animal (for example, a mouse). In the present application, researches for mice are only for illustrative purposes, unless otherwise specified, regarding the mice, also included are all non-human animals, and mice are preferred non-human animals.

In one aspect, the insertion of the human immunoglobulin heavy chain variable region gene DNA is targeted between *Tmem*121 and *Igha* genes downstream of the IgH locus on chromosome12 in mice through a site-directed recombination method; in one aspect, the human immunoglobulin heavy chain variable region gene DNA is inserted between chromosome12 position 113,149,523 and 113,223,857, properly at the position 113,190,256.

In one aspect, the insertion of the human immunoglobulin light chain variable region gene DNA is targeted between *lgkc* and *Rpia* genes downstream of the IgK locus on chromosome6 in mice through a site-directed recombination method; in one aspect, the human immunoglobulin light chain variable region gene DNA is inserted between chromosome6 position 70,703,738 and 70,742,704, properly at the position70,706,267, or at equivalent positions of λ locus chromosome 16 in mice. All the position coordinates refer to NCBI database GRCm39.

In some embodiments, the site-directed recombination method comprises a homologous recombination method, a nucleic acid cleavage enzyme mediated recombination method (such as CRISPR/Cas9), and a site-specific recombinase mediated recombination. One or more of the above methods can be combined.

In some embodiments, the constant region of the endogenous immunoglobulin heavy chain locus is completely or partially modified, so that its transcription direction is completely or partially opposite to the transcription direction of the corresponding endogenous immunoglobulin light chain variable region gene. The modification is performed by inserting exogenous recombination sites at the interior or two ends of the constant region of the non-human animal cell or animal, and then completely or partially reversing the constant region gene sequence of the host through recombinase intracellular delivery or animal breeding and other means. The insertion can be obtained by common gene editing targeting methods, such as a homologous recombination method or a nucleic acid cleavage enzyme mediated recombination method (such as CRISPR/Cas9). The modification can also be achieved through nuclease, and relevant technologies are known in the prior art.

In some embodiments, the constant region of the endogenous immunoglobulin heavy chain locus is completely or partially modified, so that its transcription direction is completely or partially opposite to the transcription direction of the corresponding endogenous immunoglobulin light chain variable region gene. The modification involves reverse insertion or replacement of a part or all of the constant region gene sequence of the host using the constant region of the non-human animal. The insertion or replacement can be obtained by common gene editing targeting methods, such as a homologous recombination method or a nucleic acid cleavage enzyme mediated recombination method (such as CRISPR/Cas9).

In some embodiments, the modified human variable region gene is first inserted downstream (in the rear) of the endogenous immunoglobulin locus of the mouse cell, then a modified short exogenous recombinase binding site is introduced in the endogenous constant region of the mouse cell, and the introduction of the recombinase makes the endogenous constant region of the mouse cell partially or completely reversed. After the obtained modified cells are screened and identified, the cells are injected into early embryos to prepare a chimeric animal (for example, a mouse). Subsequent breeding can be performed to obtain an animal containing an intact humanized immunoglobulin locus.

In some embodiments, a modified short exogenous recombinase binding site is introduced in the endogenous recombinase constant region of the mouse cell, and the introduction of the recombinase makes the endogenous recombinase constant region of the mouse cell partially or completely reversed. Then, the modified human variable region gene is inserted downstream (in the rear) of the endogenous immunoglobulin locus of the mouse cell. After the obtained modified cells are screened and identified, the cells are injected into early embryos to prepare a chimeric animal (for example, a mouse). Subsequent breeding can be performed to obtain an animal containing an intact humanized immunoglobulin locus.

Optionally, in some embodiments, a human heavy chain variable region gene can only be introduced downstream of the heavy chain locus coding region of the host non-human animal, or a human light chain variable region gene can only be introduced downstream of the light chain locus coding region of the host non-human animal, or the above two genes can be introduced simultaneously.

Not limited by any theory, the following embodiments are only intended to illustrate a fusion protein, a preparation method and use of the present application, and are not intended to limit the scope of the present invention.

### Examples

### Example 1

A mouse model of the present invention was established by inserting heavy chain genes containing complete human derived V, D and J regions downstream of the mouse immunoglobulin heavy chain coding locus and inserting the human derived light chain gene downstream of the mouse immunoglobulin light chain coding locus. Such the insertion was achieved by gene targeting in ES cells using well-known technologies in the art. A targeting vector comprising a human antibody variable region gene sequence was constructed in vitro by recombinant engineering, the targeting vector was inserted downstream of the above mouse derived immunoglobulin locus to achieve the modification of the human derived antibody variable region gene. Since the human immunoglobulin variable region gene is extremely large, a series of schemes were adopted to improve the success probability of work in order to ensure successfully, effectively and correctly obtain a mouse comprising a complete human immunoglobulin variable region. Some optional schemes are as follows.

In the present invention, a human derived gene was inserted into the Junk sequence downstream of the mouse derived immunoglobulin locus to avoid affecting the expression of the mouse derived gene in many times of gene targeting, as described in example 2 below.

Meanwhile, the operational insertion of the targeting vector designed in the present invention downstream of the mouse derived immunoglobulin gene locus can be achieved by modifying the mouse derived constant region to generate a correctly recombined chimeric antibody molecule, thereby checking the function of the inserted gene in each step in the process of modification. Please see the descriptions in examples 8 and 9 below.

When ES cell targeting was performed, in actual practice, not all the targeting vectors can be fully integrated. The ES cells were screened by high-density gene identification to obtain those cells having intact gene insertion. Such the identification can be performed by high-density PCR or Q-PCR. Through high-density PCR analysis for many times,the complete integration of the targeting vector was finally verified, and the probability of obtaining the completely integrated ES cell in the process of actual operation was about 10%. In example 3, Table 6 represents a PCR design of a partial integrity array of a first vector (vector1) in the process of heavy chain targeting.

In addition, due to the instability of the karyotype of mouse ES itself in in-vitro culture (Gaztelumendi N, Nogués C. Chromosome Instability in mouse Embryonic Stem Cells[J]. Scientific Reports, Springer Science and Business Media LLC, 2014, 4(1).), only a few cells of the obtained ES cells with intact gene insertion retain chromosome integrity and germline transmission capacity after long-term in-vitro culture and continuous targeting operations. In operation, the screened ES cells were identified by using a detailed karkyotype analysis scheme such as karyotype examination based on universal microscope inspection (photomicrographic), also such as karyotype examination based on specially designed Q-PCR (D'Hulst C, Parvanova I, Tomoiaga D, et al. Fast Quantitative Real-Time PCR-Based Screening for Common Chromosomal Aneuploidies in Mouse Embryonic Stem Cells[J]. Stem Cell Reports, Elsevier BV, 2013, 1(4): 350-359.), also such as karyotype examination based on multiple chromosomes (Barrett M T, Scheffer A, Ben-Dor A, et al. Comparative genomic hybridization using oligonucleotide microarrays and total genomic DNA[J]. Proceedings of the National Academy of Sciences, Proceedings of the National Academy of Sciences, 2004, 101(51): 17765-17770.). However, these schemes often only can reflect some abilities of the obtained ES, and cannot represent the germline transmission capacity of the obtained ES or the production ability of the human variable region chimeric antibody.

To this regard, in one feasible operation scheme of the present invention, the intermediate animals are generated and validated in a timely or timed manner (for example every 3 cell targeting modifications), and the obtained effect is that all the modified ES cells of the partial human variable region obtained by every segment of insertion can be injected into mouse embryos to produce chimeric mice. The obtained chimeric mice are all subjected to VDJ or VJ recombination by utilizing partial inserted human variable region gene, and generate the chimeric antibody with the functional human variable region. The data serves as the final check point for producing a non-human animal with intact human variable regions. From the perspective of animal production, antibody production and variable region domain diversity, it is judged that the reliability and functionality of the obtained process ES cells timely make success or failure prediction and route correction for the production of a route with an intact human variable region. These mice carrying partial human variable regions can also be used as antibody discovery for production of human variable region chimeric antibodies. Refer to data from example 8 and example 9.

### Example 2

An ES cell line of BALB/c mice was obtained by autonomous isolation, which served as a basic material for subsequent targeting.

An original vector element 1 (hereinafter referred to as Cpoint for introducing a human variable region insertion original element) was respectively knocked downstream (a region between an Igha gene and a Tmem121 gene, specifically IGH chr12:113,190,256; a region between Igkc and Rpia genes, specifically IGK chr6:70,706,267. NCBI database GRCm39) of the mouse derived IGH and IGK constant region loci in ES cells by using a well-known ES targeting technology in the art.

After a correct ES cell line was obtained, an original vector element 2 (hereinafter referred to as LD for cooperating with Cpoint) was respectively knocked upstream (a region between IgH J4 exon and Cµ locus, specifically IGH chr12: 113,391,844; a region between Igk J5 exon and Igkc gene, specifically IGK chr6: 70,701,630. NCBI database GRCm39) of the mouse derived IGH and IGK constant regions by using the well-known ES targeting technology in the art.

The above positive cell line transfected a Cre expression plasmid, and the reversion of the mouse derived C region was achieved by a Cre/loxP recombination mechanism (Zheng, B et al. "Engineering a mouse balancer chromosome." Nature genetics vol. 22,4 (1999): 375-8. doi:10.1038/11949). The above positive cell line transfected a transposase expression plasmid again, and the deletion of the screened marker fragment was achieved by a transposition mechanism (Transposase/Transposon system) (Maragathavally, K. J., et al. "Chimeric Mos1 and piggyBac transposases result in site-directed integration." The FASEB journal 20.11 (2006): 1880-1882. Wilson, Matthew H., Craig J. Coates, and Alfred L. George. "PiggyBac transposon-mediated gene transfer in human cells." Molecular therapy 15.1 (2007): 139-145.).

The targeting of the vector was performed in batches through a recombinase mediated cassette exchange (RMCE) technology (Wallace, Helen A C et al. "Manipulating the mouse genome to engineer precise functional syntenic replacements with human sequence." Cell vol. 128,1 (2007): 197-209. doi:10.1016/j.cell.2006.11.044, Prosser, Haydn M et al. "Mosaic complementation demonstrates a regulatory role for myosin VIIa in actin dynamics of stereocilia." Molecular and cellular biology vol. 28,5 (2008): 1702-12. doi:10.1128/MCB.01282-07), the deletion of the screened marker fragment was achieved by a transposition mechanism after the vector was inserted each time, until the last vector was inserted, so as to finally obtain an ES cell line where a human derived antibody variable region coding gene (VDJ) was completely inserted.

The ES cells subjected to control quality through PCR and karyotype Q-PCR array analysis were injected into the blastular cavity of the mouse according to a method in "Manipulating the Mouse Embryo: A Laboratory Manual, Fourth Edition (Cold Spring Harbor Laboratory Press, 2014)" to obtain a chimeric mouse, the obtained chimeric mouse was bred with a BALB/c mouse, and finally an IGH and IGK gene humanized mouse was obtained through genotype identification and function verification. In order to more rapidly obtain function verification data, one optional scheme is to inject ES cells into the blastaea of the mouse that cannot be subjected to functional VDJ rearrangement (such as Rag1 gene deficient mouse), and the obtained chimeric mouse can be directly subjected to immune system function verification without breeding.

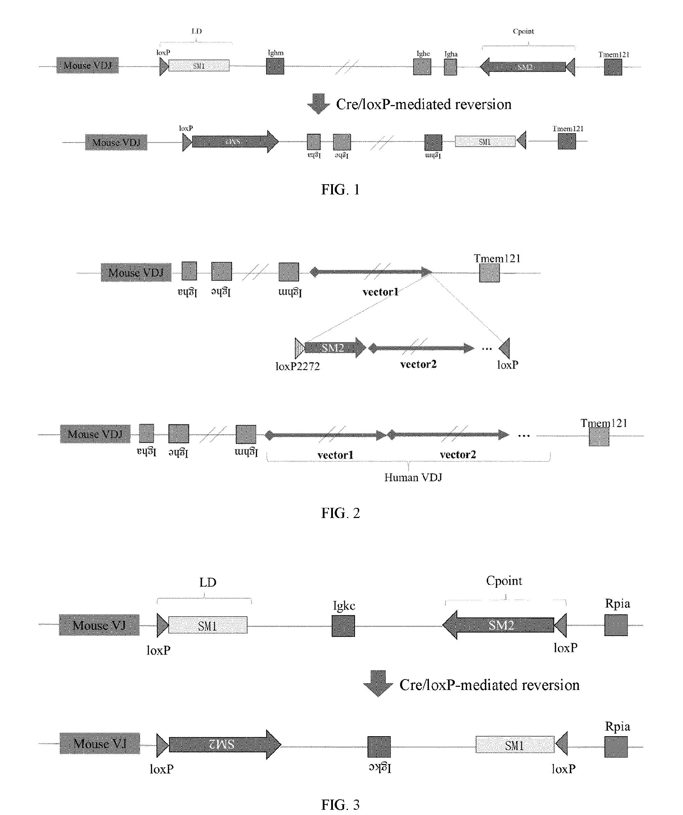

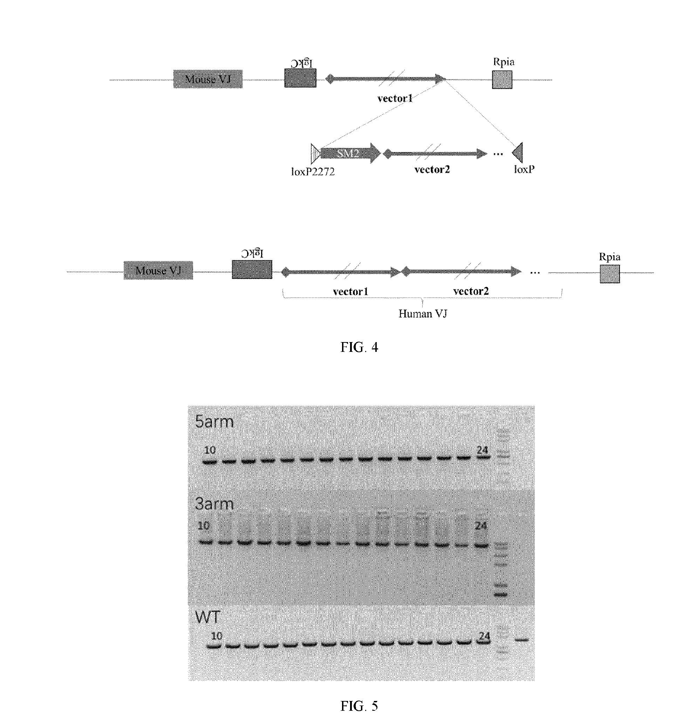

The preparation diagram and vector design are shown in FIGs. 1-4, where FIG. 1 shows Igh constant region Cpoint and LD targeting as well as C region modification; FIG. 2 is a diagram showing the insertion of the human heavy chain variable region, indicating IGH-vector targeting and resistance marker deletion; FIG. 3 shows Igk constant region Cpoint and LD targeting as well as C region modification, FIG. 4 is a diagram showing the insertion of the human light chain variable region, indicating IGK-vector targeting and resistance marker deletion.

### Example 3

### 3.1 Construction of Cpoint targeting vector

A targeting vector was composed of homologous s (sequences at two sides of an insertion site) at two ends, a screening marker (called SM for short) 2, loxP and a transposon PB fragment, as shown in FIG. 1, these fragments are linked to commercialized vector PMD18T through an enzyme linked method to construct the Cpoint targeting vector.

The Cpoint targeting vector was knocked downstream of the mouse derived IGH constant region locus by using an ES targeting technology known in the art.

It is confirmed by PCR identification that the targeting of IGH-Cpoint in clones 10-24 # was correct. Where, the Cpoint identification scheme is as shown in Table 1, and PCR identification results are as shown in FIG. 5.

**Table 1 Identification scheme of Cpoint**

| **Serial number** | **Primer name** | **Primer sequence** | **SEQ ID NO** | **Product size** |
|---|---|---|---|---|
| ①5arm | Igh-Cp-5tF 1 | actccgaggcacatgcttag | 1 | KI=756bp |
| | Igh-Cp-5tR1 | TCCTACATCGAAGCTGAAAGCACGAG | 2 | WT=/bp |
| ②3arm | Igh-Cp-3tF 1 | TACACATTTCTTCTCAAGCACTGGCTAT | 3 | KI=5805bp |
| | Igh-Cp-3tR1 | cttcgtctcttctgttatttggcgactcc | 4 | WT=/bp |
| ③ WT | Igh-Cp-wtF1 | ctgtgtagacctcaaagtcagcca | 5 | WT=1561bp |
| | Igh-Cp-wtR1 | acctccaacagttatcctgcgttaa | 6 | KI=/bp |

### 3.2 Construction of LD targeting vector

A targeting vector was composed of homologous arms (sequences at two sides of an insertion site) at two ends, a screening marker (called SM for short) 1, loxP and a transposon PB fragment, as shown in the figure, and these fragments were linked to commercialized vector PMD18T through an enzyme linked method to construct the LD targeting vector.

The ES cell with correct Cpoint targeting and the LD targeting fragment were respectively knocked upstream (a region between IgH J4 exon and Cµ locus, specifically IGH chr12: 113,391,844; a region between Igk J5 exon and Igkc gene, specifically IGK chr6: 70,701,630. NCBI database GRCm39) of the mouse derived IGH constant region.

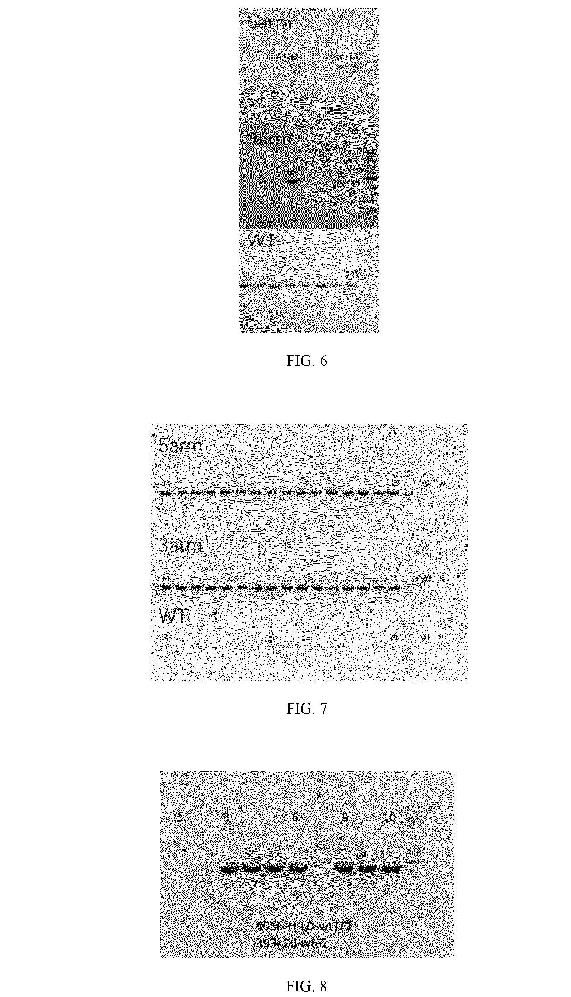

It is confirmed by PCR identification that the targeting of IGH-LD in clones 108#, 111# and 112# was correct. Where, the LD identification scheme is as shown in Table 2, and PCR identification results are as shown in FIG. 6.

**Table 2 LD identification scheme**

| **Serial number** | **Primer name** | **Primer sequence** | **SEQ ID NO** | **Product size** |
|---|---|---|---|---|
| ①5 arm | Igh-LD-5tF1 | AGGTAAGAATGGCCTCTCCAGG | 7 | KI=642bp |
| | Igh-LD-5tR1 | AAGAACCAGCCGGATCTGCAA | 8 | WT=/bp |
| ②3 arm | Igh-LD-3tF1 | CCTACCGGTGGATGTGGAAT | 9 | KI=605bp |
| | Igh-LD-3tR1 | GTGCGGAACATTCCTCACAAATC | 10 | WT=/bp |
| ③WT | Igh-LD-wtF1 | AGGTAAGAATGGCCTCTCCAGG | 7 | WT=444bp |
| | Igh-LD-wtR1 | GTGCGGAACATTCCTCACAAATC | 10 | KI=/bp |

### 3.3 Construction of Cre expression vector

A Cre expression plasmid was composed of a eukaryon promoter and a Cre coding gene as well as a terminator, and these fragments were linked to a commercialized vector PMD18T through an enzyme linked method to construct the Cre expression vector.

The ES cell with correct Cpoint and LD targeting was transfected with a Cre expression vector, and the reversion of the mouse derived C region was achieved by a Cre/loxP recombination mechanism. It is confirmed by PCR identification that the reversion of clones 14-29# was correct. Where, the reversion identification scheme is as shown in Table 3, and identification results are as shown in FIG. 7.

**Table 3 Reversion identification scheme**

| **Serial number** | **Primer name** | **Primer sequence** | **SEQ ID NO** | **Product size** |
|---|---|---|---|---|
| ①5arm | H-INV-5TR1 | ATTCAATAAACTCTTTCCCCTTGACC | 11 | KI=821bp |
| | H-INV-5TF1 | TGCTATTTGAACATAAACTAGGCCAAC | 12 | WT=/bp |
| ②3arm | H-INV-3TR1 | TAGTTGCCAGCCATCTGTTGTTTGC | 13 | KI=723bp |
| | H-INV-3TR1 | tgatgtGAgcaccttggctaccattgcttatg | 14 | WT=/bp |
| ③WT | H-INV-wtF1 | cataaacgcacaaacatacagatatatgct | 15 | WT=955bp |
| | H-INV-wtR1 | taaatggcatgatgtacgcacctt | 16 | KI=/bp |

### 3.4 Construction of transposase expression vector

A transposase expression plasmid was composed of a eukaryotic promoter and a transposase coding gene as well as a terminator, and these fragments were linked to a commercialized vector PMD18T through an enzyme linked method to construct the transposase expression vector.

A reversed positive cell line was transfected with the transposase expression plasmid, and the deletion of the screened marker fragment was achieved through a transposition mechanism.

It is confirmed by PCR identification that the deletion of resistance markers of clones 3-6# and 8-10# was correct. Where, the resistance marker deletion identification scheme is as shown in Table 4, and the identification results are as shown in FIG. 8.

**Table 4 Identification scheme for resistance marker deletion**

| **Serial number** | **Primer name** | **Primer sequence** | **SEQ ID NO** | **Product size** |
|---|---|---|---|---|
| ①FIAU | H-FIAU-tF1 | AGGTAAGAATGGCCTCTCCAGG | 7 | FIAU=644bp |
| | H-FIAU-tR1 | cactcacacataaaagttgacactggcact | 17 | |

### 3.5 Construction of IGH vector1 targeting vector

As shown in FIG. 2, through a Red/ET recombination technology (Rivero-Müller, Adolfo et al. "Assisted large fragment insertion by Red/ET-recombination (ALFIRE)--an alternative and enhanced method for large fragment recombineering." Nucleic acids research vol. 35,10 (2007): e78. doi:10.1093/nar/gkm250), a transposon, a promoter, loxP and screening marker Neo were inserted at the 5 terminal of the vector, and loxP, screening marker Puro and a transposon original element were inserted at the 3 terminal of the vector, so as to construct the IGH vector1 targeting vector.

The targeting of the human IGH vector1 was performed by an RMCE technology.

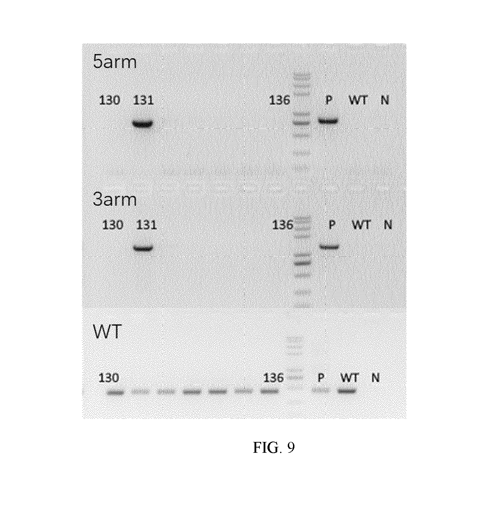

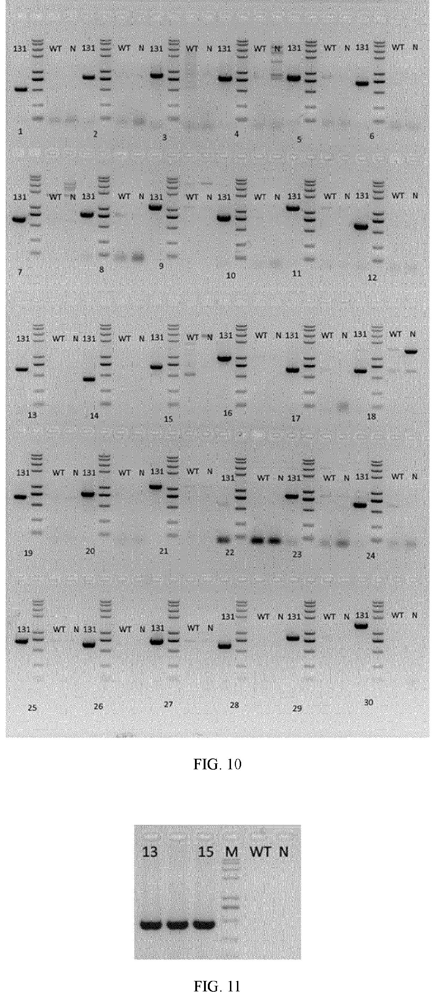

It is confirmed by PCR identification that the IGH vector1 targeting of clone 131# was correct. Where, the targeting identification scheme is as shown in Table 5, the targeting integrity identification scheme is as shown in Table 6, PCR identification results of IGH vector1 targeting are as shown in FIG. 9, and PCR identification results of IGH vector1 targeting integrity are as shown in FIG. 10.

**Table 5 Identification scheme for IGH vector1 targeting**

| **Serial number** | **Primer name** | **Primer sequence** | **SEQ ID NO** | **Product size** |
|---|---|---|---|---|
| ①5 arm | H-INV-5TR1 | ATTCAATAAACTCTTTCCCCTTGACC | 11 | KI=821bp |
| | H-INV-5TF1 | TGCTATTTGAACATAAACTAGGCCAAC | 12 | WT=/bp |
| ②3 arm | H-INV-3TR1 | TAGTTGCCAGCCATCTGTTGTTTGC | 13 | KI=723bp |
| | H-INV-3TR1 | tgatgtGAgcaccttggctaccattgcttatg | 14 | WT=/bp |
| ③WT | H-INV-wtF1 | cataaacgcacaaacatacagatatatgct | 15 | WT=955bp |
| | H-INV-wtR1 | taaatggcatgatgtacgcacctt | 16 | KI=/bp |

**Table 6 Identification scheme of IGH vector1targeting integrity**

| **Serial number** | **Detailed primer name** | **Primer sequence** | **SEQ ID NO** | **Product size (bp)** |
|---|---|---|---|---|
| 1 | H-vector-fF1 | tcttaaatccctgatcttgctcatctgtgtcg | 18 | 500 |
| | H-vector-fR1 | atgctgatgataaaaccacacagaaaataccttg | 19 | |
| 2 | H-vector-fF2 | TTAATAGGAGGCTTCCTCACAATGAAACTTTTAGAA | 20 | 769 |
| | H-vector-fR2 | GTCCCCGTCCTCACCATTCAGT | 21 | |
| 3 | H-vector-fF3 | tgacctgggtttaacctgtcctggaac | 22 | 855 |
| | H-vector-fR3 | tgacactgtggttataataactccccgaac | 23 | |
| 4 | H-vector-fF4 | gcaagggaaggctcccaaacagacga | 24 | 787 |
| | H-vector-fR4 | AAGGATGTGATCAAGTCCCTGCCGCAAA | 25 | |
| 5 | H-vector-fF5 | actgaagaacatctcctagcagcaacacc | 26 | 834 |
| | H-vector-fR5 | ttcatgtttgaaggataatttcaccagctatactgttcg | 27 | |
| 6 | H-vector-fF6 | attgacttttaaaatgcagtgtagatatgtcagag | 28 | 693 |
| | H-vector-fR6 | tcccgagtagctggaactatagatgc | 29 | |
| 7 | H-vector-fF7 | atcatgccaagcacagccgta | 30 | 600 |
| | H-vector-fR7 | ccaggagaaattccccacgtcca | 31 | |
| 8 | H-vector-fF8 | acctataaaacattaacttagaacaggggttcaattca | 32 | 763 |
| | H-vector-fR8 | ggctgattttcacagttatggaccc | 33 | |
| 9 | H-vector-fF9 | TCCCTACTGTTAAGCCACAAATCATCGTA | 34 | 1077 |
| | H-vector-fR9 | TTCCTGACCTTCTTCCCGTGTCC | 35 | |
| 10 | H-vector-fF10 | ctgtgctcagcaaagaggagaacttact | 36 | 727 |
| | H-vector-fR10 | aagacttcagagcacagactaagagc | 37 | |
| 11 | H-vector-fF11 | TGGTCTCCCATGATTCTGTTCCCAGGT | 38 | 1127 |
| | H-vector-fR11 | CAGGTGCTGAACAGATACCAGTTTCAC | 39 | |
| 12 | H-vector-fF12 | ctgtgctcagcaaagaggagaacttact | 36 | 578 |
| | H-vector-fR12 | CAGGTGCTGAACAGATACCAGTTTCAC | 39 | |
| 13 | H-vector-fF13 | tcacggagtgtctcacagtaacagtgc | 40 | 623 |
| | H-vector-fR13 | tcgagtacatttatattttgaagtcctataatcaagcctt | 41 | |
| 14 | H-vector-fF14 | agtttcttattcaagccttataattgctca | 42 | 419 |
| | H-vector-fR14 | cacatcttcttttcagatcagctcgt | 43 | |
| 15 | H-vector-fF15 | ttttatctggggtctgccgtctcc | 44 | 676 |
| | H-vector-fR15 | gctcacactgacttctccttaccgta | 45 | |
| 16 | H-vector-fF16 | GCACACTAGGCATGTCCAATTCTTACCTGG | 46 | 983 |
| | H-vector-fR16 | gctcacactgacttctccttaccgta | 45 | |
| 17 | H-vector-fF17 | gctggatgtcttagaaagagctcaaggtcctt | 47 | 639 |
| | H-vector-fR17 | aaggaaactgtatacataatgaaggactagatagg | 48 | |
| 18 | H-vector-fF18 | aatattagccaatggactatgagagtgac | 49 | 627 |
| | H-vector-fR18 | taaaagatccaaatgaaaagccaaggcaac | 50 | |
| 19 | H-vector-fF19 | tcactgagatctttacttcctttatcacgtt | 51 | 646 |
| | H-vector-fR19 | attatgttttatagtatatgtaagtgcaaactggg | 52 | |
| 20 | H-vector-fF20 | gacatgctaaagaaaatttgccaaatagcg | 53 | 772 |
| | H-vector-fR20 | aactacaggacagagataaacggccta | 54 | |
| 21 | H-vector-fF21 | ggagctaaagttgacaaataaaattgtatgtatttaaggt | 55 | 1075 |
| | H-vector-fR21 | tctgtacatctttgaggtttgccggtt | 56 | |
| 22 | H-vector-fF22 | gcacatcaacaaaataaatagacagctatccag | 57 | 562 |
| | H-vector-fR22 | agatatcgctggagcatctatatctctg | 58 | |
| 23 | H-vector-fF23 | ctgtgcatccagacccacgag | 59 | 819 |
| | H-vector-fR23 | cccctaatatcctgagtgtcccgtg | 60 | |
| 24 | H-vector-fF24 | atattcctgatgaacatagacctgaaagtcct | 61 | 617 |
| | H-vector-fR24 | gttggataggattttgttcttgattatactctca | 62 | |
| 25 | H-vector-fF25 | ctgtggctactttctcatcttaaggacga | 63 | 705 |
| | H-vector-fR25 | ggtaacaacccatcacacggctt | 64 | |
| 26 | H-vector-fF26 | gcacgtttgtctccagcacctaagtt | 65 | 626 |
| | H-vector-fR26 | tcccctggtgcatgaactct | 66 | |
| 27 | H-vector-fF27 | CATTCCAGGGCTCAAGGCAT | 67 | 716 |
| | H-vector-fR27 | GTGGCATGTCCATCTGTAGCTTGTC | 68 | |
| 28 | H-vector-fF28 | TCCACTGTGTTGTGTGTAGGCTCATCTG | 69 | 601 |
| | H-vector-fR28 | GTGGCATGTCCATCTGTAGCTTGTC | 68 | |
| 29 | H-vector-fF29 | caggtatttctttacaattcaataatggacga | 70 | 823 |
| | H-vector-fR29 | cacagatagattgagaattttccaagcctt | 71 | |
| 30 | H-vector-fF30 | gatgttgtattagtccattctcacggtgct | 72 | 1364 |
| | H-vector-fR30 | agccaagaaataaagacaagtttctacaaccac | 73 | |

IGH vector1 targeting positive cells were transfected with a transposase expression plasmid, and the deletion of the screened marker fragment was achieved by a transposition mechanism. It is confirmed by PCR identification that the deletion of resistance markers of clones 13-15# was correct. Where, the resistance marker deletion identification scheme is as shown in Table 7, and the PCR identification results of IGH vector1 resistance marker deletion are as shown in FIG. 11.

**Table 7 Identification scheme for resistance marker deletion**

| **Serial number** | **Primer name** | **Primer sequence** | **SEQ ID NO** | **Product size** |
|---|---|---|---|---|
| ①FIAU | H-vector1-FIAU-tF1 | AACAAACCAGGGGTCTTAGTGATG | 74 | FIAU=48 5bp |
| | H-vector1-FIAU-tR1 | GGCTAGATGCCTTTCTCCCTTGAC | 75 | |

Clones in which IGH vector1-FIAU targeting was correct were subjected to quality control of karyotype Q-PCR array analysis. The results show that the clones meet quality control standard.

The ES cells subjected to quality control through PCR and karyotype Q-PCR array analysis were injected into the blastular cavity of the mouse according to a method in "Mouse Embryo Operation Experiment Manual" to obtain a F0 chimeric mouse, the F0 chimeric mouse was bred with a BALB/c mouse, and a humanized mouse with partial human IGH gene was finally contained through genotype identification and function verification.

### Example 4

### The humanized mouse can correctly use human variable region genes

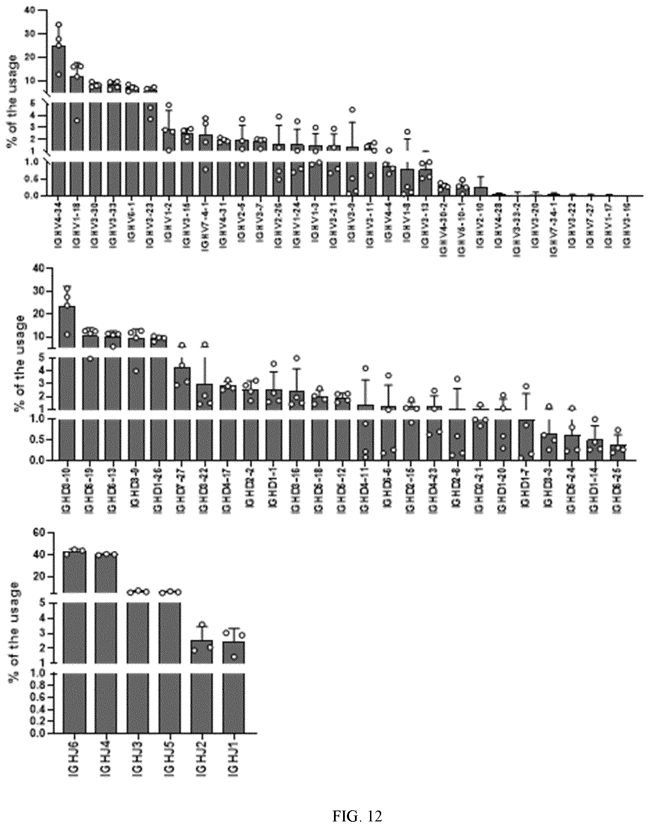

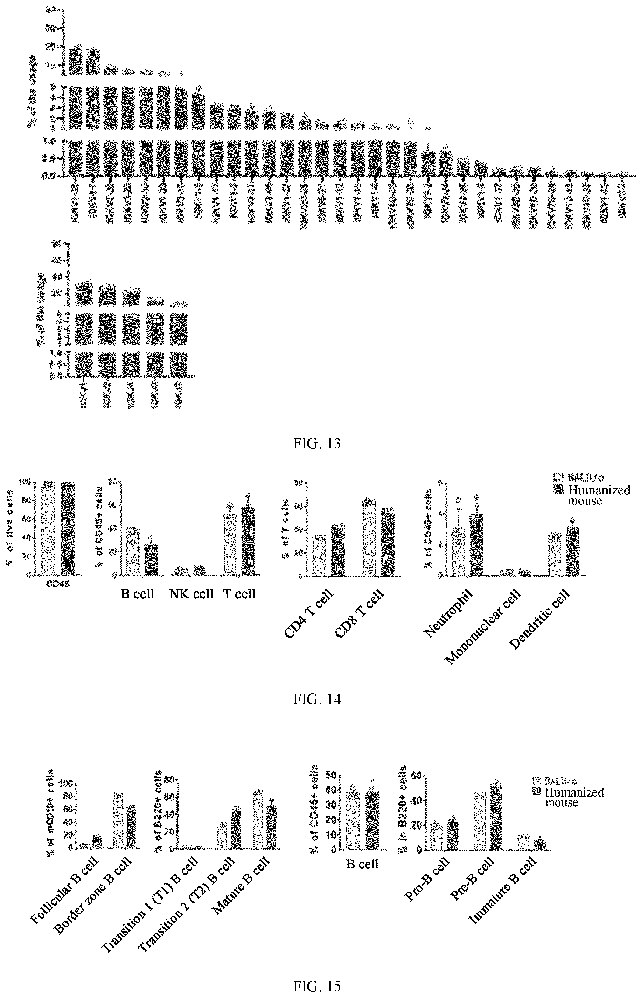

The spleen of the humanized mouse was collected, and the availability of the utilization of inserted human genes was assessed through BCR sequencing in spleen B cells. The BCR sequencing results are as shown in FIG. 12 and FIG. 13, proving that the humanized mouse can correctly use human IGH and IGK variable region genes.

### Example 5

### The development of immune cells of humanized mouse was correct

The development of immune related cells, especially B cells, in the spleen and bone marrow of the humanized mouse was detected by flow cytometry. The results are as shown in FIG. 14 and FIG. 15, proving that the proportions of lymphocytes and myeloid cells in the spleen and bone marrow are normal, and the development of the B cells in the spleen and bone marrow is correct.

### Example 6

### The reversion of a constant region reduces the leakage expression of mouse derived genes

In the actual use of the humanized mouse, the problem of the leakage expression of the mouse derived VDJ genes may occur. The existing technical scheme aims at the inactivation of mouse derived variable region genes, mainly including the following two types: 1. the mouse derived variable region genes were completely deleted, but a large number of other endogenous genes in mice were deleted at the same time, damaging endogenous genes or transcriptional regulatory elements; 2. the mouse derived variable region genes were reversed so that they are in opposite direction, but the mouse endogenous variable region genes were closely adjacent to the inserted human variable region genes. In practice, the direction of the V gene in the Igk locus of the mouse is diverse, where also included is IGKV gene whose direction is the same as or opposite to that of the J and C genes of respective IGK (Collins, Andrew M., and Corey T. Watson. "Immunoglobulin light chain gene rearrangements, receptor editing and the development of a self-tolerant antibody repertoire." Frontiers in immunology 9 (2018): 2249.). The reversed variable region is still closely adjacent to the human variable region, and both of them are still located upstream of the endogenous constant region (5' terminal) in spatial position. From the principle and direction of genome transcription translation, there is still a risk of mouse derived variable regions participating in the V(D)J recombination process.

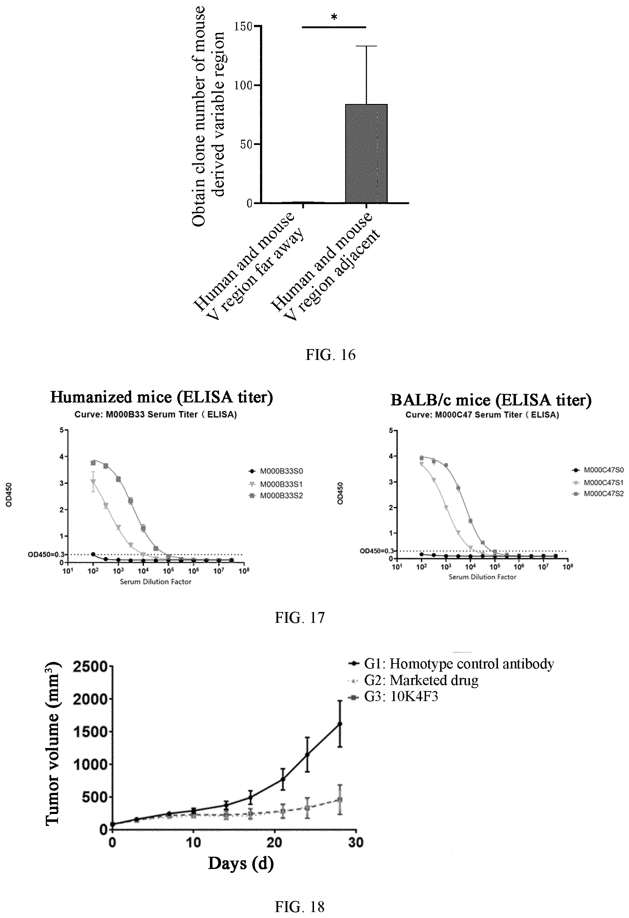

In this patent, the human variable region genes were inserted downstream of the mouse derived immunoglobulin locus, and the endogenous variable region is separated from the inserted human variable region by using the endogenous constant region. Comparison is made between mice in which the endogenous variable region is distanced from the inserted human variable region, as disclosed in this patent, and mice in existing technologies where the endogenous variable region is adjacent to the inserted human variable region. BCR sequencing is performed on the spleens of both sets of mice. The abundance of variable region sequences with higher homology to mouse is detected to confirm the involvement of mouse-origin variable regions in VDJ recombination usage. As shown in FIG. 16, the leakage expression of the mouse derived variable region VDJ rearrangement in the scheme that human-mouse variable regions are closely adjacent is detected many times, and the leakage expression in the scheme that human-mouse variable regions far away from the mouse derived variable regions is almost not detected. In this patent, the effect obtained by inserting the human variable region gene downstream of the immunoglobulin genome achieves a complete alteration of the relative positioning of human variable regions and mouse variable regions, and the problem of the leakage expression of the mouse derived variable region participating in VDJ gene rearrangement is solved.

### Example 7

### A humanized mouse can generate high-affinity antibodies

The humanized mouse was immunized by using a commercialized hPD1 recombinant protein, and the ELISA titer detection of the serum antibody was performed. The results show that the immune responses of the humanized mouse and the BALB/c mouse to the same PD1 antigen are equivalent. A positive antibody 10K4F3 was obtained through a hybridoma fusion screening technology, and the affinity (positive control uses BMK, which was a marketed antibody drug) of the antibody was measured through surface plasmon resonance. The efficacy of the antibody was evaluated by using a mouse in-vivo efficacy experiment,

The results are as shown in Table 8, FIG. 17 and FIG. 18, proving that the use of the humanized mouse can generate the high-affinity antibody, and the corresponding antibody has good in-vivo anti-tumor efficacy data.

**Table 8 Affinity comparison table of screened 10K4F3 antibody and marketed drug**

| Cloning | Kd (1/s) | KD (M) |
|---|---|---|
| 10K4F3 | 1.24E-04 | 6.78E-10 |
| BMK-positive antibody | 2.85E-03 | 8.81E-09 |

### Example 8

### Preparation of partial variable region gene humanized mouse

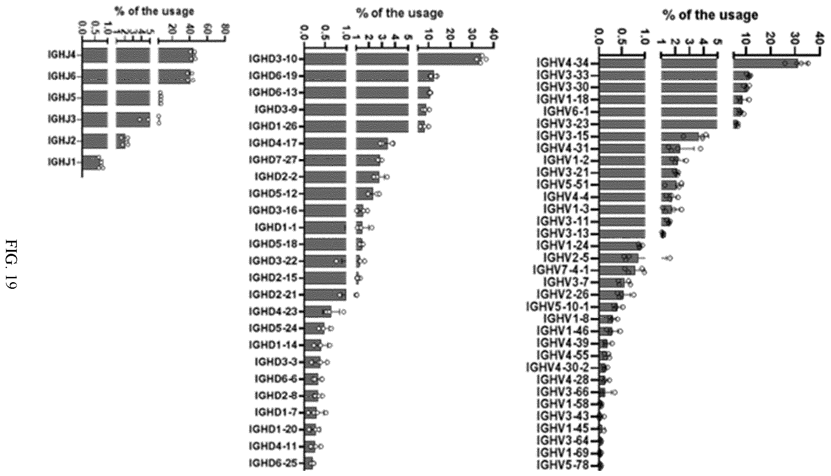

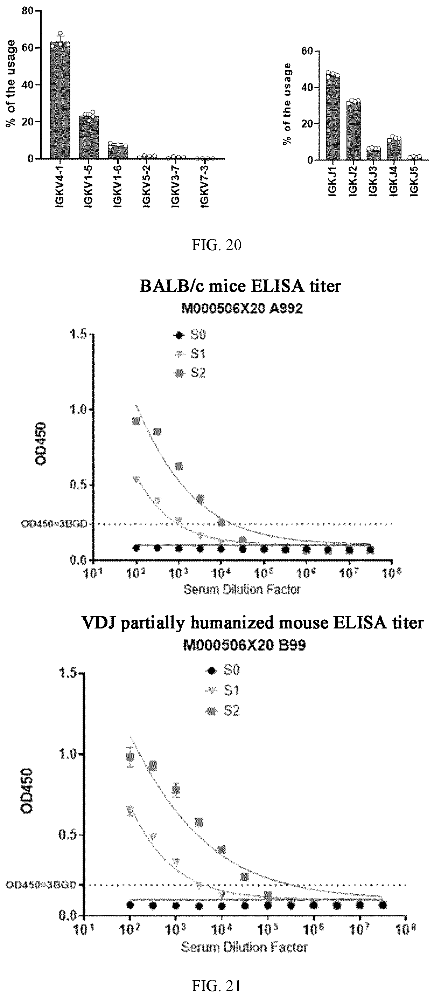

Partial human derived IGH-V, D and J genes and partial human derived IGK-V and J genes (specific gene names are list in FIG. 19 and FIG. 20) were used to obtain a humanized mouse with partial VDJ genes through the same method. The spleen of the positive mouse was collected, the availability of the utilization of inserted human genes was assessed through BCR sequencing in spleen B cell. The BCR sequencing results are as shown in FIG. 19 and FIG. 20, proving that even though partial VDJ genes are inserted, the humanized mouse still can use this portion of human variable region gene.

### Example 9

### Ahumanized mouse with partial variable region genes can correctly generate immune response

A commercialized OVA protein was used to immunize a VDJ partially humanized mouse, and underwent serum antibody ELISA titer detection. The results are as shown in FIG. 21, showing that the responses of the humanized mouse and the BALB/c mouse to the OVA antigen are equivalent.

## Claims

1. A method for preparing a non-human animal, the method comprising operably linking a human immunoglobulin variable region gene downstream of an immunoglobulin locus of the non-human animal.

2. The method according to claim 1, comprising operably linking a human immunoglobulin heavy chain variable region gene downstream of an immunoglobulin heavy chainlocus of the non-human animal.

3. The method according to any one of claims 1-2, comprising operably linking genes of one or more human heavy chain variable V regions, human heavy chain variable D regions or human heavy chain variable J regions, or fragments thereof downstream of the heavy chain constant region locus of the non-human animal.

4. The method according to claim 3, wherein the genes of the more human heavy chain variable V regions, human heavy chain variable D regions or human heavy chain variable J regions, or fragments thereof are directly linked.

5. The method according to claim 1, the method comprising operably linking a human immunoglobulin light chain variable region gene downstream of a light chain locus of the non-human animal.

6. The method according to claim 5, comprising operably linking the genes of one or more human light chain variable V or J regions, or fragments thereof downstream of a light chain constant region locus of the non-human animal.

7. The method according to claim 6, wherein the genes of the more human light chain variable V regions or human light chain variable J regions, or fragments thereof are directly linked.

8. The method according to any one of claims 1-7, whereof the non-human animal comprises endogenous immunoglobulin variable region genes.

9. The method according to any one of claims 1-8, wherein the integrity of the endogenous immunoglobulin variable region genome of the non-human animal is not changed.

10. The method according to any one of claims 1-9, wherein the function of the expression regulatory element of the endogenous immunoglobulin variable region gene of the non-human animal is not damaged.

11. The method according to any one of claims 1-10, whereof the non-human animal comprises the intact expression regulatory element of the endogenous immunoglobulin variable region.

12. The method according to any one of claims 1-11, whereof the non-human animal comprises intact endogenous immunoglobulin variable region genes.

13. The method according to any one of claims 1-12, whereof the non-human animal does not express the endogenous immunoglobulin variable region.

14. The method according to any one of claims 1-13, wherein the endogenous immunoglobulin variable region genes of the non-human animal do not express functional proteins.

15. The method according to any one of claims 1-14, comprising allowing the transcription direction of the human immunoglobulin variable region gene to be opposite to that of the endogenous immunoglobulin variable region gene.

16. The method according to any one of claims 1-15, comprising allowing the transcription direction of the immunoglobulin constant region gene of the non-human animal to be opposite to that of the endogenous immunoglobulin variable region gene.

17. The method according to any one of claims 1-16, wherein the transcription direction of the human immunoglobulin variable region gene of the non-human animal is the same as that of the immunoglobulin constant region gene of the non-human animal.

18. The method according to any one of claims 1-17, wherein a heavy chain constant region gene of the non-human animal is contained between the human immunoglobulin heavy chain variable region gene and the endogenous immunoglobulin heavy chain variable region gene of the non-human animal.

19. The method according to any one of claims 1-18, wherein a distance between the human immunoglobulin heavy chain variable region gene and the endogenous immunoglobulin heavy chain variable region gene of the non-human animal is 169 Kbp-240 Kbp.

20. The method according to any one of claims 1-19, wherein a light chain constant region gene of the non-human animal is contained between the human immunoglobulin light chain variable region gene and the endogenous immunoglobulin light chain variable region gene of the non-human animal.

21. The method according to any one of claims 1-20, wherein a distance between the human immunoglobulin light chain variable region gene and the endogenous immunoglobulin light chain variable region gene of the non-human animal is 4 Kbp-42 Kbp.

22. The method according to any one of claims 1-21, whereof the non-human animal is a rodent.

23. The method according to any one of claims 1-22, whereof the non-human animal is a mouse.

24. The method according to claim 23, comprising inserting the human immunoglobulin heavy chain variable region gene between *Tmem121* and *Igha* genes in the mouse locus.

25. The method according to any one of claims 23-24, comprising inserting the human immunoglobulin heavy chain variable region gene between mouse chromosome positions chr12:113,149,523-113,223,857.

26. The method according to any one of claims 23-25, comprising inserting the human immunoglobulin heavy chain variable region gene at the mouse chromosome position chr12: 113,190,256.

27. The method according to any one of claims 23-26, comprising inserting the human immunoglobulin light chain variable region gene between *lgkc* and *Rpia* genes in the mouse locus.

28. The method according to any one of claims 23-27, comprising inserting the human immunoglobulin light chain variable region gene between mouse chromosome positions chr6: 70,703,738-70,742,704.

29. The method according to any one of claims 23-28, comprising inserting the human immunoglobulin light chain variable region gene at the mouse chromosome position chr6: 70,706,267.

30. The method according to any one of claims 1-29, comprising operably linking the human immunoglobulin variable region gene downstream of the immunoglobulin locus of the non-human animal through site-directed recombination.

31. The method according to any one of claims 1-30, comprising allowing the transcription direction of the immunoglobulin constant region gene of the non-human animal to be opposite to that of the endogenous immunoglobulin variable region gene through genome modification.

32. A non-human animal whose genome comprises a human immunoglobulin variable region gene operably linked downstream of the immunoglobulin locus.

33. The non-human animal according to claim 32, comprising a human immunoglobulin heavy chain variable region gene operably linked downstream of the heavy chain locus.

34. The non-human animal according to any one of claims 32-33, comprising genes of one or more human heavy chain variable V regions, human heavy chain variable D regions or human heavy chain variable J regions, or fragments thereof operably linked downstream of the heavy chain constant region locus..

35. The non-human animal according to any one of claims 32-34, wherein the genes of the more human heavy chain variable V regions, human heavy chain variable D regions or human heavy chain variable J regions, or fragments thereof are directly linked.

36. The non-human animal according to claim 32, comprising a human immunoglobulin light chain variable region gene operably linked downstream of the light chain locus.

37. The non-human animal according to claim 36, comprising genes of one or more human light chain variable V regions, or human light chain variable J regions, or fragments thereof operably linked downstream of the light chain constant region locus.

38. The non-human animal according to any one of claims 36-37, wherein the genes of the more human light chain variable V regions or human light chain variable J regions, or fragments thereof are directly linked.

39. The non-human animal according to any one of claims 32-38, comprising endogenous immunoglobulin variable region genes.

40. The non-human animal according to any one of claims 32-39, wherein the integrity of the endogenous immunoglobulin variable region genome is not changed.

41. The non-human animal according to any one of claims 32-40, wherein the function of the expression regulatory element of the endogenous immunoglobulin variable region gene is not damaged.

42. The non-human animal according to any one of claims 32-41, comprising the intact expression regulatory element of the endogenous immunoglobulin variable region.

43. The non-human animal according to any one of claims 32-42, comprising intact endogenous immunoglobulin variable region genes.

44. The non-human animal according to any one of claims 32-43, whereof the non-human animal does not express the endogenous immunoglobulin variable region.

45. The non-human animal according to any one of claims 32-44, wherein the endogenous immunoglobulin variable region gene of the non-human animal does not express a functional protein.

46. The non-human animal according to any one of claims 32-45, wherein the transcription direction of the human immunoglobulin variable region gene of the non-human animal genome is opposite to that of the endogenous immunoglobulin variable region gene.

47. The non-human animal according to any one of claims 32-46, wherein the transcription direction of the immunoglobulin constant region gene of the non-human animal genome is opposite to that of the endogenous immunoglobulin variable region gene.

48. The non-human animal according to any one of claims 32-47, wherein the transcription direction of the human immunoglobulin variable region gene is the same as that of the immunoglobulin constant region gene of the non-human animal.

49. The non-human animal according to any one of claims 32-48, wherein the heavy chain constant region gene is contained between the human immunoglobulin heavy chain variable region gene and the endogenous immunoglobulin heavy chain variable region gene.

50. The non-human animal according to any one of claims 32-49, wherein a distance between the human immunoglobulin heavy chain variable region gene and the endogenous immunoglobulin heavy chain variable region gene is 169 Kbp-240 Kbp.

51. The non-human animal according to any one of claims 32-50, wherein the light chain constant region gene is contained between the human immunoglobulin light chain variable region gene and the endogenous immunoglobulin light chain variable region gene.

52. The non-human animal according to any one of claims 32-51, wherein a distance between the human immunoglobulin light chain variable region gene and the endogenous immunoglobulin light chain variable region gene is 4 Kbp-42 Kbp.

53. The non-human animal according to any one of claims 32-52, whereof the non-human animal is a rodent.

54. The non-human animal according to any one of claims 32-53, whereof the non-human animal is a mouse.

55. The non-human animal according to claim 54, wherein the human immunoglobulin heavy chain variable region gene is contained between *Tmem121* and *Igha* genes in the locus.

56. The non-human animal according to any one of claims 54-55, wherein the human immunoglobulin heavy chain variable region gene is contained between thechromosomal positions chr12: 113,149,523-113,223,857

57. The non-human animal according to any one of claims 54-56, wherein the human immunoglobulin heavy chain variable region gene is contained at chromosomal position chr12: 113,190,256.

58. The non-human animal according to any one of claims 54-57, wherein the human immunoglobulin light chain variable region gene is inserted between *lgkc* and *Rpia* genes in the locus.

59. The non-human animal according to any one of claims 54-58, wherein human immunoglobulin variable light region gene is inserted between chromosome positions chr6:70,703,738-70,742,704.

60. The non-human animal according to any one of claims 54-59, wherein the human immunoglobulin variable light region gene is inserted at chromosome position chr6:70,706,267.

61. A non-human animal cell, wherein the genome of the non-human animal cell comprises human immunoglobulin variable region gene downstream of the immunoglobulin locus.

62. A method for preparing an antibody specifically binding to an antigen, the method comprising immunizing the non-human animal according to any one of claims 32-60 with the antigen.

63. A method for preparing a sample, the method comprising: exposing the non-human animal according to any one of claims 32-60 to an antigen; and collecting the sample from the non-human animal.

64. The method according to claim 63, wherein the sample comprises an immune cell.

65. The method according to claim 63 or 64, wherein the sample is a B cell.

66. The method according to any one of claims 63-65, wherein the sample is derived from bone marrow, spleen tissues, lymph nodes, spleen cells or peripheral lymphocytes.
